Europäisches Patentamt

European Patent Office

Office européen des brevets

(11) Publication number: **0 078 828**
**B1**

(12) **EUROPEAN PATENT SPECIFICATION**

(45) Date of publication of patent specification: **16.04.86**

(21) Application number: **82901565.0**

(22) Date of filing: **13.05.82**

(86) International application number:
**PCT/SE82/00170**

(87) International publication number:
**WO 82/04127 25.11.82 Gazette 82/28**

(51) Int. Cl.⁴: **G 01 N 1/20**

(54) **APPARATUS FOR SAMPLING OF LIQUID.**

(30) Priority: **15.05.81 SE 8103066**

(43) Date of publication of application:
**18.05.83 Bulletin 83/20**

(45) Publication of the grant of the patent:
**16.04.86 Bulletin 86/16**

(84) Designated Contracting States:
**DE FR GB NL**

(56) References cited:
**DE-B-2 312 010**
**SE-A-8 008 285**
**US-A-3 253 469**
**US-A-4 022 066**

(73) Proprietor: **Arla, ekonomisk förening**
**Dalagatan 3**
**S-105 46 Stockholm (SE)**

(72) Inventor: **OLENFALK, Lars**
**Planetvägen 3**
**S-175 60 Järfälla (SE)**

(74) Representative: **Stürmer, Gerhard et al**
**AWAPATENT AB Box 7402**
**S-103 91 Stockholm (SE)**

## Description

The invention relates to an apparatus for sampling of liquid when the liquid is being led through a conduit or the like. The invention has primarily been developed for sampling of milk when milk from the milk producer is collected by a milk-lorry, and sampling is to be carried out for chemical and bacteriological analysis.

Practice has shown that the milk can become different in different layers in the milk producer's tank even if this incorporates an agitator. As the milk fat and protein content is decisive for the price and the milk is allowed to have only a certain maximum bacterial content, samples must be taken for chemical and bacteriological analysis. However, due to the stratification of the milk problems arise during sampling. Similar problems can arise in other conjunctions and therefore the use of this apparatus is by no means restricted to the dairy industry. This apparatus can, on the contrary, be used everywhere where samples are to be taken from a liquid in the cases where the liquid is led through a conduit and when it is desired to take a sample which is representative of the entire liquid quantity.

• To solve the above-mentioned problem one has hitherto had to choose between using rather costly sampling equipments to be provided in each milk-lorry or taking in a vessel sample quantities which in total have been relatively large (5—10 liters) and, after stirring, withdrawing therefrom a minor quantity in a sampling bottle from analysis. In the latter case it is always one and the same vessel that is used from farm to farm and this involves a risk of bacterial infections and also bacterial growth in the sampling vessel and, consequently, misleading analysis results. In Sweden milk samples from each supplier are still taken by hand, with a ladle, many times a month, which may amount to 100 000 samples for one and the same dairy in the course of one year.

The object of the present invention is therefore to provide an improved sampling apparatus of the type described above, which is simple and worth its price, which eliminates the above inconveniences and which permits a representative sampling covering each delivery from the various suppliers, while the sample quantity from each delivery is to be constantly small and the risk of infection from the environment must be at a minimum. Moreover, the sampling apparatus must be easily washed up and disinfected.

This object is realized according to this invention as disclosed in claim 1.

US—A—4,022,066 shows an apparatus for sampling of liquid when the liquid is being led through a conduit having arranged therein a volume meter and a pump and also a sampling valve controlled by supplied pulses from a pulse converter for complete opening or complete closing of a communication between the liquid conduit and a sample vessel. The sampling valve has a cylindrical bore and communicates with the liquid conduit via an opening, said bore including a longitudinally movable sampling piston which is adapted to be actuated by a unit controlled by the impulses from the pulse converter to open and close the communication between the opening and the sample vessel in response to these incoming impulses.

In this apparatus use is made of a pulse generator for sampling controlled by a time generator, i.e. a certain number of milliliter samples are taken after a certain number of seconds, independently of the amount of the flow and depending only upon the present setting of the time generator. Furthermore this apparatus has not the characteristic features that the flow meter or the pump emits, depending on the flow rate of liquid passing through said flow meter or pump, a pulse to the pulse converter which in turn is adapted to emit one impulse per predetermined number of incoming pulses, and that the pulse converter is adapted to receive data of the predetermined liquid volume unit from which the total sampling volume is to be taken and that the pulse converter is adapted to adjust the length and/or amplitude of each pulse emitted by the pulse converter in such a manner that the sampling apparatus gives one and the same total sampling volume from the sampling valve to the sample vessel in response to each predetermined liquid volume unit which passes through the flow meter and/or the pump.

US—A—3,253,469 describes an apparatus in which the sample taken is always directly proportional to the flow. This means that 1 liter samples are taken e.g. from 1000 liter flow while 3 liter samples are taken from 3000 liter flow, etc. According to the present invention, however, e.g. 1000 ml samples are taken from X liter flow independently of whether X corresponds to e.g. 50, 500 or 5000 liter or, practically speaking, any amount whatsoever.

The invention will now be described more fully with reference to an example of embodiment shown in the accompanying drawing. In the drawing:

Fig. 1 shows a milk tank to which a milk-lorry is coupled for collection of milk and simultaneous sampling during emptying of the tank; and

Fig. 2 shows a section through an embodiment of the sampling valve used in this connection.

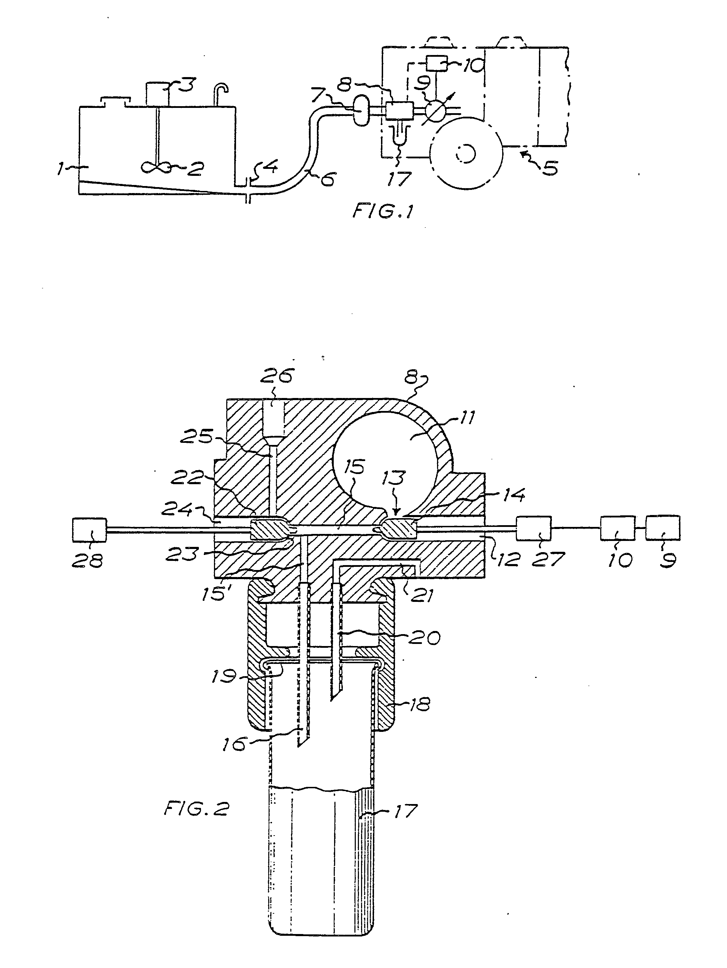

Referring now to Fig. 1, the milk producer's tank 1 is assumed to be provided with an agitator 2 driven by a motor 3. At the bottom on one side of the tank there is a joint 4 for connection of the tap hose 6 of the milk-lorry 5.

The tap hose 6 leads to a pump 7. This pump is preferably designed so as always to permit pumping the same quantity during each revolution of the pump, irrespectively of the suction height — which, however, hardly exceeds 2—3 m w.c. — and counterpressure. Such pumps are called positive pumps, they close backwards like a non-return valve and at a constant speed they produce a constant flow.

After the pump 7 the milk first passes a sampling valve 8, which will be further described below with reference to Fig. 2, and then a flow meter 9 before landing in one of the tanks (not shown) of the milk-lorry 5. The flow meter 9 is adapted to emit an impulse to an impulse converter 10 in response to each volume unit of milk passing through said meter. After a certain number of such impulses, which number may be fixed or be chosen with respect to the milk quantity contained in the tank 1 and in that case is set on a transmitter to the impulse converter, a sampling pulse is supplied to the sampling valve 8. The length and/or the amplitude of this sampling pulse is variable or adjustable so that the liquid volume, which as a rule is known in advance, through the flow meter will give one and the same total sampling volume delivered by the sampling valve. In this way it will be possible, for instance from a tank holding 1000 l milk, to take 100 l ml milk samples, thus giving a sample quantity totalling 0.1 l in which the sample is representative and is easy to handle.

An embodiment of the sampling valve 8 according to the invention is shown in section in Fig. 2. Through the valve 8 a milk conduit extends in the form of a channel 11 situated between the pump 7 and the flow meter 9. At the right the bottom of the channel 11 is tangent to a cylindrical bore 12 which, via an opening 13, communicates with said channel 11 and accommodates a sampling valve piston 14. The sampling valve piston 14 is actuated by an electrical control unit 27 which is controlled by impulses delivered by the impulse converter 10 on the basis of the count pulses of the flow meter 9. In Fig. 2 the units 27, 10 and 9 have only been indicated by squares and are chosen by the expert within the field of prior art.

According to its position the sampling valve piston 14 can thus, more or less and for a shorter or longer time, open or close the communication between the opening 13 and a sampling channel 15, 15' which downwardly opens into a hollow tap needle 16. The tap needle 16 is run through the cover of a plastic bottle 17 when this is inserted from below into the sample vessel holder 18. A hollow venting needle 20, placed beside the tap needle 16, penetrates the cover 19 at the same time. The venting needle 20 communicates via a venting channel 21 in the sampling valve housing with the outer atmosphere or possibly with a source of negative pressure.

The sampling channel, which comprises a horizontal section 15 and a vertical section 15', communicates, at the transition between these sections 15 and 15', with a valve seat 23 for a cleaning valve piston 22 which is movable in a second cylindrical bore 24 by means of a control unit 28. If the cleaning valve piston 22 is withdrawn — i.e. to the left in Fig. 2 — the sampling channel 14, 15' is brought in communication with a cleaning and disinfection channel 25 which, at a connection 26, may be supplied with a suitable liquid for cleaning or disinfection and can be supplied with compressed air for blowing clean the channel sections 15 and 15', the tap needle 16 and the opening 13.

The sampling apparatus according to the invention, such as illustrated in Figs. 1 and 2 — operates in the following manner when the milk-lorry 5 via the tap hose 6 has been connected to the milk supplier's tank 1.

The pump 7 is started after the impulse converter 10 has been set for the milk quantity concerned, and for a short time the tap hose 6, the pump 7, the sampling valve 8 etc. are "flushed clear" from milk residues from the preceding delivery by new milk. This is followed by sampling in the sample receiving vessel 17 which has previously been inserted into the sample vessel holder 18 and has already been provided with the client's number, date, hour, milk-lorry number etc. Say that a quantity of about 3 200 l is to be pumped over to the milk lorry. A small sample is to be taken for every 10th litre and the total sample quantity is to be 100 ml. This results in about 320 samples of about 0.30 ml. The impulse length and the impulse amplitude, the pulse number and, consequently the opening stroke length, the opening time and the opening frequency of the sampling valve piston 14 are automatically calculated out by feeding the impulse converter with the intended milk quantity.

As long as the pump 7 is running and milk passes through the flow meter 9 the latter emits pulses to the pulse converter 10 in which they are processed and which emits pulses to the this control unit 27 of the sampling valve piston 14, which unit opens and closes the opening 13 at a regular rate, at a certain frequency and stroke length so that small splashes of milk are continuously supplied, via the channels 15, 15' and the tap needle 16, to the sample vessel 17 which is successively filled and will contain about 100 ml when the tank 1 is emptied. The flow of milk from the milk conduit 11 to the sample vessel takes place due to the pressure difference therebetween, by positive pressure in the milk conduit 11 or negative pressure in the venting channel 21 or both.

According to a further development of the inventive concept the tap needle 16 and the venting needle 20 serve as electrodes for a level control, e.g. in the form of a measuring bridge where the electrodes are included in the measuring branch. If the milk rises in the sampling vessel to such a level that also the venting needle will come in contact with the milk, then the electrical resistance between the needles and the electrodes 16 and 20 will change, which acts upon the measuring bridge and is used to close the sampling valve piston 14 or to stop the pump 7. Repletion of the sampling vessel and contamination due to outflowing milk is thereby avoided. This may be of great value if the driver due to an oversight has forgotten to set the calculated quantity of milk or set it for too small a quantity. The sampling vessel will never overflow and if the pump 7 has been stopped it is only necessary to take away the filled sampling vessel and replace it by another sampling vessel, whereupon the pumping-over operation can continue and continued samples can be taken. It will thus be possible to avoid both repletion of the sampling vessel and errors in sampling. The only possible inconvenience is that the sampling quantity obtained may be greater than what is normally the case. In total, however, the samples are always representative.

Total cleaning and disinfection of the sampling valve takes place in connection with cleaning and disinfection of those other parts of the milk-lorry which come in contact with the milk. As to the sampling valve the sampling valve piston 14 is first opened repeatedly, wherein the sampling valve is flushed by cleaning and disinfection liquid from the milk channel 11. Furthermore, cleaning and disinfection liquid is supplied, via the connection 26 and the channel 25, to the cleaning piston 22 which is pulled back by the control unit 28, whereupon it is possible to blow clean with compressed air which is supplied at 26 and empties the sampling channel 15, 15' and the tap needle 16.

The advantages of the sampling apparatus according to the invention may be summarized as follows. The sample obtained is always representative of the entire flow quantity. Normally the sample quantity obtained is always practically the same independently of the liquid amount passing the flow meter. The sample is not distorted by the preceding delivery and can be restricted to a relatively small and easily manageable volume. There is no risk of clogging in the sampling valve and the possibility of cleaning and sterilization is simple because the valve has its own internal washing and disinfection circuit whereby no milk residues will be standing in the valve channels. The whole apparatus will be quite worth its price and its build-up is simple and uncomplicated. Moreover, it will be easy to provide the apparatus with a level control, and the space requirements for the entire sampling apparatus are very small. Of course, the apparatus herein described by way of example can be used not only in milk-lorries but also stationarily for sampling in dairies and also for sampling of other liquids than milk.

## Claims

1. Apparatus for sampling liquid when the liquid is being led through a conduit (11) which has arranged therein (11) a flow meter (9) and/or a positive pump (7) and a sampling valve (8) which valve is controlled by pulses supplied from a pulse converter (10) for opening and closing a communication (13—16) between the conduit (11) and a sample vessel (17), said sampling valve (8) including a cylindrical bore (12) and communicating via an opening (13) with the conduit (11), said bore (12) including a longitudinally movable sampling piston (14) which is adapted to be actuated by a unit (27) controlled by the impulses from the pulse converter (10) to open and close the opening (13), characterized in that the cylindrical bore (12) is generally tangential to the conduit (11), that the flow meter (9) or the pump (7) is adapted to emit, dependent on the flow rate of liquid passing through said flow meter or pump, pulses to the pulse converter (10) which in turn is adapted to emit one impulse per predetermined number of incoming pulses from the flow meter (9) or the pump (7), respectively, and in that the pulse converter (10) is adapted to receive data of the predetermined liquid volume unit from which the total sampling volume is to be taken and in that the pulse converter (10) is adapted to adjust the length and/or amplitude of each pulse delivered by said pulse converter (10) in such a manner that the sampling apparatus gives one and the same total sampling volume from the sampling valve (8) to the sample vessel (17) in response to each predetermined liquid volume unit which passes through the flow meter (9) and/or the pump (7).

2. Apparatus as claimed in claim 1, characterized in that a second cylindrical bore (24) in the housing of the sampling valve (8) includes a cleaning valve piston (22) which is actuated by a control unit (28) and cooperates with a valve seat (23) and wherein the valve seat (23) can connect a sampling channel (15, 15') with a feed line (25, 26) for cleaning and disinfection liquid or compressed air for clean-blowing.

3. Apparatus as claimed in claim 1 or 2, characterized in that the communication between the opening (13) and the sample vessel (17) consists of a sampling channel (15, 15') and a hollow tap needle (16).

4. Apparatus as claimed in claim 1, 2, or 3, characterized in that the sampling valve (8) at its bottom has a sample vessel holder (18) for the sample vessel (17), the sample vessel is provided with a thin plastic cover (19) and wherein there is a second hollow needle, situated beside the hollow tap needle (16), for venting the sample vessel (17) and/or for application of low pressure to the sample vessel.

5. Apparatus as claimed in claim 4, characterized in that the tap needle (16) and the venting needle (20) are connected as electrodes to a level control which prevents the sample vessel (17) from being supplied with more liquid than it can hold.

6. Apparatus as claimed in any of the preceding claims, characterized in that the pump (7), the sampling valve (8) with the sample vessel holder (18), the flow meter (9) and the impulse converter (10) are combined into a unit in a milk-lorry or a dairy.

## Revendications

1. Appareil d'échantillonnage de liquide pour prélever des échantillons d'un liquide amené à s'écouler à travers une conduite (11), dans laquelle sont installés un débitmètre (9) et/ou une pompe volumétrique (7) en une soupape d'échantillonnage (8), laquelle est commandée par des impulsions fournies par un convertisseur d'impulsions (10) en vue de l'ouverture et de la fermeture d'une communication (13—16) entre la conduite (11) et un récipient à échantillons (17), la soupape d'échantillonnage (8) possédant un alésage cylindrique (12) et communiquant avec la conduite (11) à travers un orifice (13), l'alésage (12) contenant un piston d'échantillonnage (14) déplaçable longitudinalement et qui est actionné par une unité (27) commandée par les impulsions venant du convertisseur d'impulsions (10) en vue de l'ouverture et de la fermeture de l'orifice (13), caractérisé en ce que l'alésage cylindrique (12) est généralement tangentiel à la conduite (11), que le débitmètre (9) ou la pompe (7) est conçu pour délivrer des impulsions en fonction du débit de liquide traversant le débitmètre ou la pompe au convertisseur d'impulsions (10), fournissant lui-même chaque fois une impulsion pour un nombre prédéterminé d'impulsions entrantes provenant du débitmètre (9) ou de la pompe (7), que le convertisseur d'impulsions (10) est conçu pour recevoir des informations concernant le volume de liquide, prédéterminé, d'où doit être prélevé le volume total des échantillons, formant ensemble un échantillon du volume de liquide prédéterminé, et que le convertisseur d'impulsions (10) est conçu pour ajuster la longueur et/ou l'amplitude de chaque impulsion fournie par lui, de manière que l'appareil d'échantillonnage délivre toujours le même volume total d'échantillons par sa soupape d'échantillonnage (8) au récipient à échantillons (17) pour chaque volume de liquide prédéterminé traversant le débit-mètre (9) et/ou la pompe (7).

2. Appareil selon la revendication 1, caractérisé en ce qu'un second alésage cylindrique (24) dans le corps de la soupape d'échantillonnage (8) contient un piston de nettoyage (22) qui est actionné par une unité de commande (28) et qui coopère avec un siège (23), et en ce que le siège (23), lorsqu'il est découvert par le piston, relie un canal d'échantillonnage (15, 15') à une canalisation d'alimentation (25, 26) pour du liquide de nettoyage et de désinfection ou pour de l'air comprimé pour le nettoyage par soufflage.

3. Appareil selon la revendication 1 ou 2, caractérisé en ce que la communication entre l'orifice (13) et le récipient à échantillons (17) est formé d'un canal d'échantillonnage (15, 15') et d'une aiguille creuse de soutirage (16).

4. Appareil selon la revendication 1, 2 ou 3, caractérisé en ce que la soupape d'échantillonnage (8) présente à son fond un support (18) pour le récipient à échantillons (17), lequel récipient est pourvu d'un mince couvercle (19) en plastique, et en ce que l'appareil comporte une seconde aiguille creuse, située à côté de l'aiguille creuse de soutirage (16) et destinée à l'échappement d'air du récipient à échantillons (17) et/ou à l'application d'une dépression à l'intérieur de ce récipient.

5. Appareil selon la revendication 4, caractérisé en ce que l'aiguille de soutirage (16) et l'aiguille d'échappement d'air (20) sont branchées à la façon d'électrodes à un dispositif de limitation de niveau qui empêche l'introduction d'une trop grande quantité de liquide dans le récipient à échantillons (17).

6. Appareil selon l'une quelconque des revendications précédentes, caractérisé en ce que la pompe (7), la soupape d'échantillonage (8) avec le support pour (18) le récipient à échantillons, le débitmètre (9) et le convertisseur d'impulsions (10) sont combinés en une unité dans un camion-citerne de ramassage de lait ou dans une laiterie.

**Patentansprüche**

1. Vorrichtung zur Probenahme aus einer durch eine Leitung (11) o.dgl. strömenden Flüssigkeit, wobei in der Leitung (11) ein Durchflussmesser (9) und/oder eine positive Pumpe (7) sowie ein Probenahmeventil (8) angeordnet sind, welches durch von einem Pulswandler (10) zugeführte Pulse zwecks Öffnen und Schliessen einer Verbindung (13—16) zwischen der Leitung (11) und einem Probegefäss (17) gesteuert wird, wobei das Probenahmeventil (8) eine zylindrische Bohrung (12) aufweist und über eine Öffnung (13) in Verbindung mit der Leitung (11) steht, wobei in der Bohrung (12) ein in deren Längsrichtung verschiebbarer Probenahmekolben (14) sitzt, der durch eine von den Impulsen des Pulswandlers (10) gesteuerte Vorrichtung (27) zum Öffnen und Schliessen der Öffnung (13) beaufschlagt wird, dadurch gekennzeichnet, dass die zylindrische Bohrung (12) die Flüssigkeitsleitung (11) tangiert, dass der Durchflussmesser (9) und/oder die Pumpe (7) für jede durchströmende Flüssigkeitsvolumeneinheit einen Puls an den Pulswandler (10) abgibt, welcher seinerseits einen Impuls pro fest vorgebbare Anzahl hereinkommender Pulse vom Durchflussmesser (9) bzw. der Pumpe (7) abgibt, sowie dass die Länge und/oder Amplitude jeden vom Impulswandler (10) abgegebenen Pulses so variierbar bzw. einstellbar ist, dass die Probenahmevorrichtung für jedes am Impulswandler (10) einstellbare, den Durchflussmesser (9) bzw. die Pumpe (7) durchströmende Flüssigkeitsvolumen ein und dasselbe gesamte Probenahmevolumen vom Probenahmeventil (8) zum Probenahmegefäss (17) ergibt.

2. Vorrichtung nach Anspruch 1, dadurch gekennzeichnet, dass in einer zweiten zylindrischen Bohrung (24) im Gehäuse des Probenahmeventils (8) ein Reinigungsventilkolben (22) sitzt, der von einer Betätigungsvorrichtung (28) beaufschlagbar ist und mit einem Ventilsitz (23) zusammenarbeitet, sowie dass der Ventilsitz (23) einen Probenahmekanal (15, 15') mit einer Zuleitung (25, 26) für Säuberungs- und Desinfektionsflüssigkeit oder Druckluft zum Sauberblasen verbinden kann.

3. Vorrichtung nach Anspruch 1 oder 2, dadurch gekennzeichnet, dass die Verbindung zwischen der Öffnung (13) und dem Probenahmegefäss (17) aus einem Probenahmekanal (15, 15') sowie einer hohlen Zapfnadel (16) besteht.

4. Vorrichtung nach Anspruch 1, 2 oder 3, dadurch gekennzeichnet, dass unten am Probenahmeventil (8) ein Probegefässhalter (18) für das Probenahmegefäss (17) sitzt, dass das Probenahmegefäss mit einem dünnen Kunstoffdeckel (19) versehen ist, sowie dass neben der hohlen Zapfnadel (16) eine zweite Hohlnadel (20) zur Entlüftung des Probenahmegefässes (17) und/oder Zufuhr von Unterdruck zum Probenahmegefäss sitzt.

5. Vorrichtung nach Anspruch 4, dadurch gekennzeichnet, dass die Zapfnadel (16) und Entlüftungsnadel (20) an Elektroden einer Ueberfüllungsschutzvorrichtung angeschlossen sind, welche verhindert, dass dem Probenahmegefäss (17) mehr Flüssigkeit zugeführt wird als dieses aufnehmen kann.

6. Vorrichtung nach einem der vorstehenden Patentansprüche, dadurch gekennzeichnet, dass die Pumpe (7), das Probenahmeventil (8) mit dem Probegefässhalter (18), Durchflussmesser (9) und dem Pulswandler (10) zu einer einzigen Einheit in einem Milchauto oder einer Molkerei verbunden sind.

0 078 828

FIG.1

FIG.2

1